# EUROPEAN PATENT APPLICATION

(11) **EP 4 772 275 A1**
(43) Date of publication of application: **08.07.2026**
(21) Application number: 24859765.0
(22) Date of filing: 27.08.2024
(51) Int. Cl.: B01J 23/75, C01B 3/00, C07B 61/00, C07C 4/06, C07C 9/02, C07C 9/14

(54) **METAL CATALYST AND METHOD FOR HYDROGENOLYSIS OF CYCLIC COMPOUND USING SAME**

(30) Priority: 01.09.2023 JP 2023142495
(71) Applicant: Chiyoda Corporation, Kanagawa 220-8765 (JP); NATIONAL UNIVERSITY CORPORATION YOKOHAMA NATIONAL UNIVERSITY, Yokohama-shi Kanagawa 240-8501 (JP)
(72) Inventor: KUBOTA, Yoshihiro, Yokohama-shi, Kanagawa 240-8501 (JP); INAGAKI, Satoshi, Yokohama-shi, Kanagawa 240-8501 (JP); MAEKAWA, Yuki, Yokohama-shi, Kanagawa 240-8501 (JP); WAKATSUKI, Rikuto, Yokohama-shi, Kanagawa 240-8501 (JP); HODOSHIMA, Shinya, Yokohama-shi, Kanagawa 220-8765 (JP); IMAGAWA, Kenichi, Yokohama-shi, Kanagawa 220-8765 (JP)
(74) Representative: Ipsilon Luxembourg
(86) International application number: PCT/JP2024/030484
(87) International publication number: WO 2025/047733

(57) **Abstract**

[Problem] To selectively hydrocrack a cyclic compound that has a five-membered ring structure and is included in a saturated cyclic compound used as a hydrogen carrier.

[Solution] A metal catalyst used for hydrocracking a cyclic compound that has a five-membered ring structure and is included in a saturated cyclic compound used as a hydrogen carrier, comprises a silica-based support serving as a catalyst carrier, and cobalt as a metal supported on the catalyst carrier.

## Description

### TECHNICAL FIELD

The present invention relates to a metal catalyst used for hydrocracking a cyclic compound having a five-membered ring structure and a method for hydrocracking a cyclic compound using the same.

### BACKGROUND ART

Known methods for storing and transporting hydrogen include the organic chemical hydride method (OCH Method; Non-Patent Documents 1 and 2). The organic chemical hydride method involves a hydrogenation reaction (i.e., hydrogen storage reaction) in which an aromatic compound(s) such as toluene react with hydrogen, and a dehydrogenation reaction (i.e., hydrogen generation reaction) in which hydrogen is generated from a saturated cyclic compound(s) (i.e., hydrogenated aromatic compound) such as methylcyclohexane to recover the aromatic compound(s) such as toluene. In the organic chemical hydride method, a hydrogen-absorbed saturated cyclic compound can be stored and transported in a liquid state at room temperature and atmospheric pressure, and a required amount of hydrogen can be extracted through the dehydrogenation reaction of the saturated cyclic compound at a site where hydrogen is to be utilized. A saturated cyclic compound such as methylcyclohexane and an aromatic compound such as toluene, which are produced after hydrogen is extracted from the saturated cyclic compound, are repeatedly used as containers for hydrogen (i.e., hydrogen carriers).

It has been well known that hydrogenation and dehydrogenation reactions in the organic chemical hydride method produce various by-products (impurities) other than target products (Patent Document 1).

### PATENT DOCUMENT(S)

[Patent Document 1]
JP2007-269522A

### NON-PATENT DOCUMENT(S)

[Non-Patent Document 1]
   Yoshimi Okada, Energy and Resources, Vol. 33, No. 3, 168 (2018)
[Non-Patent Document 2]
   Yoshimi Okada, Tokyo High Pressure Gas Association Bulletin, August and September 2019
[Non-Patent Document 3]
   Xieli Sai, Mika Ishii, Tetsuya Namba, Taku Tsujimura, Takaaki Taniguchi, 46th Petroleum and Petrochemical Symposium, Lecture Abstract, 2A03 (2016)

### SUMMARY OF THE INVENTION

### TASK TO BE ACCOMPLISHED BY THE INVENTION

Thus, in the organic chemical hydride method, repeated hydrogenation and dehydrogenation reactions gradually increase the rate of impurities in the hydrogen carrier. One possible approach to addressing this problem is to distill the hydrogen carrier containing impurities to thereby remove (separate) such impurities from the hydrogen carrier.

In particular, impurities that can be mixed into the hydrogen carrier include cyclic compounds with a five-membered ring structure (herein also referred to as "five-membered ring compounds") such as cyclopentanes, which are produced in the dehydrogenation reaction (Non-Patent Document 3). Some of five-membered ring compounds, in particular, those with seven carbon atoms (such as dimethylcyclopentanes and ethylcyclopentanes), generally have their boiling points that are relatively close to the boiling point of the hydrogen carrier, which makes it difficult to separate these five-membered ring compounds from the hydrogen carrier by distillation.

As a result of efforts to address this problem, the inventors have developed a metal catalyst used for selectively hydrocracking five-membered ring compounds mixed in the hydrogen carrier, and found that use of the metal catalyst enables the conversion of the five-membered ring compounds in the hydrogen carrier into hydrocracked light hydrocarbons.

The present invention has been made based on the above-described problem of the prior art, and a primary object of the present invention is to provide a metal catalyst used for hydrocracking a cyclic compound that has a five-membered ring structure and is included in a saturated cyclic compound used as a hydrogen carrier, and a method for hydrocracking such a cyclic compound using the same.

### MEANS TO ACCOMPLISH THE TASK

As a solution to the above-described tasks to be accomplished, an aspect of the present invention provides a metal catalyst used for hydrocracking a cyclic compound that has a five-membered ring structure and is included in a saturated cyclic compound used as a hydrogen carrier, the metal catalyst comprising: a silica-based support serving as a catalyst carrier, and cobalt as a metal supported on the catalyst carrier.

This configuration enables the selective hydrocracking of a cyclic compound having a five-membered ring structure included in a saturated cyclic compound used as a hydrogen carrier. In other words, in a process of hydrocracking a cyclic compound having a five-membered ring structure mixed in a hydrogen carrier, this configuration enables the proper hydrocracking of the cyclic compound while minimizing the loss of the hydrogen carrier (i.e., unintended chemical reaction).

The above metal catalyst may be further configured such that the metal catalyst comprises cobalt as the metal in an amount ranging from 1 wt% to 50 wt%.

This configuration, in which a proper amount of cobalt is supported on the catalyst carrier, enables the stable hydrocracking of the cyclic compound having the five-membered ring structure.

The above metal catalyst may be further configured such that the metal catalyst comprises cobalt as the metal in an amount of 5 wt% or more.

This configuration can effectively increase the selectivity toward lighter hydrocarbons with fewer carbon atoms (e.g., C₁-C₅ alkanes).

The above metal catalyst may be further configured such that the silica-based support comprises amorphous silica.

This configuration can effectively increase the selectivity toward lighter hydrocarbons with fewer carbon atoms (e.g., C₁-C₅ alkanes).

As a solution to the above-described tasks to be accomplished, another aspect of the present invention provides a method for hydrocracking a cyclic compound using the above metal catalyst, wherein the cyclic compound has a five-membered ring structure and is included in a saturated cyclic compound used as a hydrogen carrier, the method comprising: causing dehydrogenation of the saturated cyclic compound to generate an aromatic compound and the cyclic compound having the five-membered ring structure, as an impurity of the aromatic compound; causing hydrogenation of the aromatic compound containing the impurity, thereby generating a mixture of the saturated cyclic compound and the impurity; and hydrocracking the mixture of the saturated cyclic compound and the impurity using the metal catalyst.

In a system where the organic chemical hydride method is carried out, this configuration enables the selective hydrocracking of a cyclic compound having a five-membered ring structure included in a saturated cyclic compound used as a hydrogen carrier.

The above method may be further configured such that the step of hydrocracking comprises: supplying the mixture of the saturated cyclic compound and the impurity together with 50 to 99.8 mol% hydrogen to the metal catalyst having a catalyst layer, while a temperature of the catalyst layer is maintained at 150°C to 300°C.

This configuration causes the hydrocracking of the mixture of the saturated cyclic compound and the impurity together with 50 to 99.8 mol% hydrogen at a proper temperature to enable the stable hydrocracking of the cyclic compound having the five-membered ring structure.

### EFFECT OF THE INVENTION

The present invention embodied as described above, enables the selective hydrocracking of a cyclic compound having a five-membered ring structure included in a saturated cyclic compound used as a hydrogen carrier.

### BRIEF DESCRIPTION OF THE DRAWINGS

[Figure 1] Figure 1 is a block diagram showing a schematic configuration of a hydrogen storage and transportation system according to one embodiment of the present invention;
[Figure 2] Figure 2 is a diagram showing by-products in the dehydrogenation reaction of methylcyclohexane and their boiling points;
[Figure 3] Figure 3 is an explanatory diagram showing the hydrocracking (hydrogenolysis) of methylcyclopentane;
[Figure 4] Figure 4 is an explanatory diagram showing the distribution of reaction products in the hydrocracking of MCP;
[Figure 5] Figure 5 is an explanatory diagram showing the distribution of reaction products in the hydrocracking of MCH; and
[Figure 6] Figure 6 is an explanatory diagram showing the distribution of reaction products in the hydrocracking of a mixture of MCP and MCH.

### DESCRIPTION OF THE PREFERRED EMBODIMENT(S)

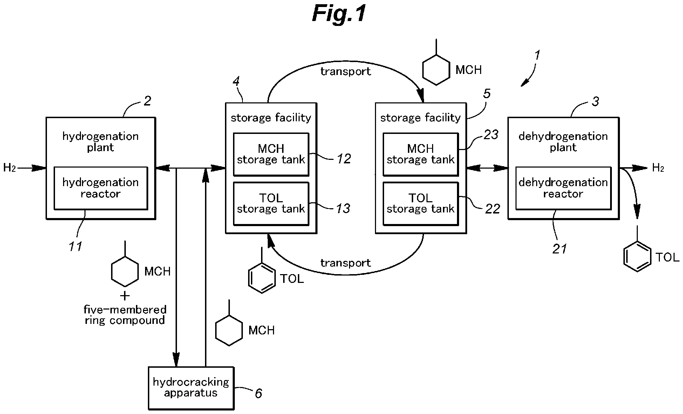

A metal catalyst and a method for hydrocracking a cyclic compound having a five-membered ring structure (herein also referred to as "five-membered ring compound(s)") using the metal catalyst according to an embodiment of the present invention will be described with reference to the appended drawings. In embodiments described below, a metal catalyst and a method for hydrocracking a compound using the same according to the present invention are embodied in a hydrogen storage and transportation system in which an organic chemical hydride method is carried out, as shown in Figure 1.

The hydrogen storage and transportation system 1 includes a hydrogenation plant 2 that generates saturated cyclic compounds as organic hydrides by adding hydrogen to aromatic compounds, and a dehydrogenation plant 3 that generates hydrogen and aromatic compounds by dehydrogenating the saturated cyclic compounds. In the following example, a saturated cyclic compound to be used is methylcyclohexane (hereinafter also referred to as "MCH" as necessary), and a corresponding aromatic compound to be used is toluene (hereinafter also referred to as "TOL" as necessary).

The hydrogenation plant 2 is provided with a hydrogenation reactor 11 that produces methylcyclohexane by causing a hydrogenation reaction to add hydrogen (H₂) to toluene, using a known method. The system includes a storage facility 4 associated with the hydrogenation plant 2. The storage facility 4 includes an MCH storage tank 12 for storing the methylcyclohexane produced by the hydrogenation reactor 11. The storage facility 4 also includes a TOL storage tank 13 for storing toluene transported from the dehydrogenation plant 3 (in a storage facility 5).

The dehydrogenation plant 3 is provided with a dehydrogenation reactor 21 that produces hydrogen and toluene by causing a dehydrogenation reaction of methylcyclohexane, using a known method. The system includes the storage facility 5 associated with the dehydrogenation plant 3. The storage facility 5 includes a TOL storage tank 22 for storing the toluene produced by the dehydrogenation reactor 21. The storage facility 5 also includes an MCH storage tank 23 for storing the methylcyclohexane transported from the hydrogenation plant 2 (in the storage facility 4).

In the hydrogen storage and transportation system 1, the methylcyclohexane stored in the MCH storage tank 12 is transported to the dehydrogenation plant 3 using a known means for transportation (such as ship, vehicle, or pipeline) and then stored in the MCH storage tank 23. The methylcyclohexane stored in the MCH storage tank 23 is used in a dehydrogenation reaction in the dehydrogenation reactor 21. The toluene stored in the toluene storage tank 22 is transported to the hydrogenation plant 2 using a known means for transportation and stored in the toluene storage tank 13. The toluene stored in the toluene storage tank 13 is used in the hydrogenation reaction in the hydrogenation reactor 11. In this way, the methylcyclohexane and toluene are repeatedly used as containers for hydrogen (hydrogen carriers).

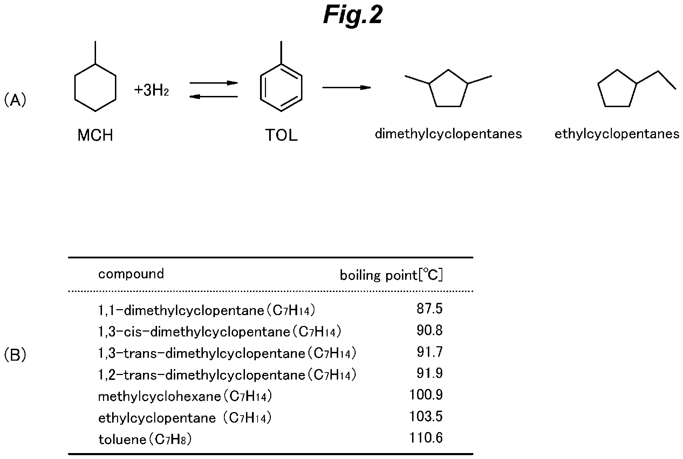

As shown in Figure 2(A), the dehydrogenation reaction in the dehydrogenation reactor 21 generates, in addition to hydrogen and toluene, by-products containing five-membered ring compounds. Such five-membered ring compounds include dimethylcyclopentanes (e.g., 1,1-dimethylcyclopentane, 1,3-cis-dimethylcyclopentane, 1,3-trans-dimethylcyclopentane, and 1,2-trans-dimethylcyclopentane) and ethylcyclopentane. The boiling points of the five-membered ring compounds are relatively close to those of methylcyclohexane and toluene, which are used as hydrogen carriers, as shown in Figure 2(B), which makes it difficult to separate the five-membered ring compounds from the hydrogen carrier through distillation.

The five-membered ring compounds produced in dehydrogenation reactor 21 are mixed with toluene and stored in the TOL storage tank 22, and then transported to the hydrogenation plant 2 via the TOL storage tank 13. Thus, the methylcyclohexane produced in the hydrogenation plant 2 may contain the five-membered ring compounds derived from the above-described dehydrogenation reaction as impurities. The five-membered ring compounds mixed with methylcyclohexane, are transported to the dehydrogenation plant 3 again, and thus circulate within the hydrogen storage and transportation system 1. The five-membered ring compounds can be produced not only by the dehydrogenation reaction in the dehydrogenation reactor 21, but also by the hydrogenation reaction in the hydrogenation reactor 11.

Hence, the hydrogen storage and transportation system 1 further includes a hydrocracking apparatus 6, which uses a metal catalyst (hereinafter referred to as the "hydrocracking catalyst") to selectively hydrocrack five-membered ring compounds that are present as impurities in methylcyclohexane, thereby converting the five-membered ring compounds into chain light hydrocarbons.

The hydrocracking apparatus 6 extracts at least part of the products of the hydrogenation reaction (primarily methylcyclohexane, e.g., 98 wt% or more) generated in the hydrogenation plant 2 as a material to be processed before the extracted products are stored in the storage facility 4, and causes a hydrocracking reaction. In the hydrocracking apparatus 6, five-membered ring compounds included in the products of the hydrogenation reaction are decomposed into lighter hydrocarbons (e.g., pentane, hexane, and heptane). In particular, the five-membered ring compounds can be decomposed into lighter hydrocarbons with fewer carbon atoms (e.g., C₁-C₅ alkanes) using a properly selected metal as the hydrocracking catalyst. The obtained hydrocarbons can be removed (i.e., separated) from methylcyclohexane by distillation or any other known suitable process. Methylcyclohexane, from which the five-membered ring compounds have been removed (or reduced), is reintroduced into the product stream of the hydrogenation plant 2.

The material to be processed by the hydrocracking apparatus 6 may be methylcyclohexane (containing the five-membered ring compounds as impurities) extracted from the MCH storage tank 12 of the storage facility 4. In that case, the methylcyclohexane, from which the five-membered ring compounds have been removed by the hydrocracking apparatus 6, is returned to the MCH storage tank 12. In some cases, the hydrocracking apparatus 6 may be connected to a feed supply line (not shown) which is further connected to the dehydrogenation reactor 21 that forms a part of the dehydrogenation plant 3.

The material to be processed by the hydrocracking apparatus 6 may be methylcyclohexane (containing the five-membered ring compounds as impurities) extracted from the MCH storage tank 23 of the storage facility 5. In that case, the methylcyclohexane, from which the five-membered ring compounds have been removed by the hydrocracking apparatus 6, is returned to the MCH storage tank 23. In some cases, the hydrocracking apparatus 6 may be connected to a product discharge line (not shown) which is further connected to the hydrogenation reactor 11 that forms a part of the hydrogenation plant 2.

For example, a fixed-bed circulating reactor is used as the hydrocracking apparatus 6. The hydrocracking apparatus 6 includes a temperature controller (e.g., an electric heater or a heat exchanger using a heat medium). Preferably, when the hydrocracking reaction occurs in the hydrocracking apparatus 6, the temperature of a catalyst layer comprising the hydrocracking catalyst is maintained within a range of 150°C to 300°C by the temperature controller. The metal catalyst of the present embodiment exhibits good catalytic performance even when the temperature of the catalyst layer is 200°C or lower. Examples of reaction feedstock used in the hydrocracking reaction may include a mixture of toluene and five-membered ring compounds, along with 50-99.8 mol% hydrogen.

Examples of hydrocracking catalysts used in the hydrocracking apparatus 6 include hydrocracking catalysts comprising silica as a catalyst carrier and cobalt as a metal supported on the catalyst carrier. The hydrocracking catalyst may contain 1 to 50 wt% (weight percent) cobalt. More preferably, the hydrocracking catalyst may contain 5 wt% or more cobalt. Amorphous silica may be used as the silica-based carrier for the catalyst carrier. However, the silica-based carrier is not limited to amorphous silica and may be, for example, pure silica zeolites such as Silicalite-1, Zeolite Beta, and high-silica USY (ultra-stable Y), or ordered mesoporous silicas such as FSM-16, MCM-41, MCM-48, and SBA-15.

The organic hydrides used in the hydrogen storage and transportation system 1 can be any organic compound that reversibly releases hydrogen through a catalytic reaction, and are not limited to the methylcyclohexane as described above. The organic hydrides that can be used in the hydrogen storage and transportation system 1 may include a monocyclic hydrogenated aromatic compound such as methylcyclohexane or cyclohexane, a bicyclic hydrogenated aromatic compound such as tetralin, decalin, or methyl decalin, and a tricyclic hydrogenated aromatic compound such as tetradecahydroanthracene either alone or in combination. When the organic hydride is changed, another aromatic compound corresponding thereto produced by a dehydrogenation reaction is used in place of toluene.

The hydrocracking catalysts and methods for hydrocracking a five-membered ring compound using the same according to preferred embodiments of the present invention will be described with reference to Examples 1-4 and Comparative Example 1.

The above-described hydrocracking apparatus 6 is configured to be used to hydrocrack five-membered ring compounds introduced as impurities into the products of the hydrogenation reaction in the hydrogenation plant 2 (or generated as byproducts of the reaction) using a hydrocracking catalyst. A reaction feedstock (FS) (i.e., material to undergo the hydrocracking reaction) in the hydrocracking apparatus 6 is a mixture of methylcyclohexane and five-membered ring compounds generated by the hydrogenation reaction of toluene. In Examples 1-3 and Comparative Example 1, methylcyclopentane ("MCP") was used as a model compound for the five-membered ring compound contained (i.e., mixed) in the reaction feedstock. Furthermore, in Example 4, a mixed feedstock of MCP and MCH ("MCP+MCH") was used to make the conditions more practical.

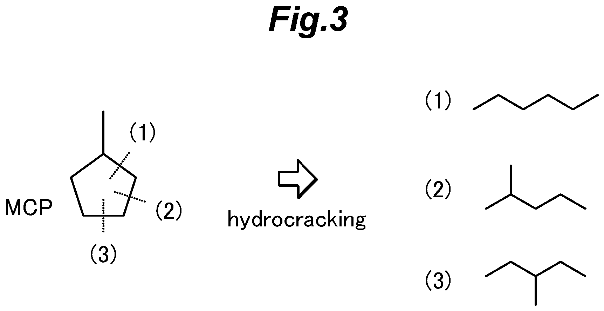

For example, as shown in Figure 3, the hydrocracking of methylcyclopentane (MCP) can cause MCP ring to open at positions (1) to (3), yielding chain compounds corresponding to the opening position (such as hexane, 2-methylpentane, or 3-methylpentane). Furthermore, these linear compounds can undergo further decomposition to produce C₁-C₅ alkanes. Unlike five-membered ring compounds, the products from the hydrocracking reaction can be readily separated from methylcyclohexane by distillation or any other known suitable process. Similarly, the products from the hydrocracking reaction of ethylcyclopentane and other five-membered ring compounds can also be readily separated from methylcyclohexane.

Table 1 indicates catalyst samples used in Examples 1-4 and Comparative Example 1. As described in detail below, Catalysts used in Examples 1-4 (Ex 1-4) are those in which cobalt (Co) is supported on an amorphous silica catalyst carrier at different loading levels. A catalyst used in Comparative Example 1 (C. Ex 1) is in which a commercially available catalyst sample wherein platinum (Pt) supported on a carrier, as described below.

**[Table 1]**

| | catalyst | metal loading rate [wt%] | support |
|---|---|---|---|
| C. Ex. 1 | 1.0wt%-Pt/SiO₂ | 1.0 | commercialized prod |
| Ex. 1 | 3.0wt%-Co/SiO₂ | 3.0 | amorphous silica |
| Ex. 2 | 5.0wt%-Co/SiO₂ | 5.0 | amorphous silica |
| Ex. 3 & 4 | 10wt%-Co/SiO₂ | 10.0 | amorphous silica |

Table 2 indicates reaction feedstock used in Examples 1-4 and Comparative Example 1.

**[Table 2]**

| feedstock (FS) | MCP+H 2 (C. Ex. 1 and Ex. 1-3) | MCH+H2 (C. Ex. 1 and Ex. 1-3) | (MCP+MCH)+H2 (Ex. 4) |
|---|---|---|---|
| H2/MCP rate [mol/mol] | 28 | - | - |
| H2/MCH rate [mol/mol] | - | 28 | - |
| H2/(MCP+MCH) rate [mol/mol] | - | - | 28 |

In Examples 1-3 and Comparative Example 1, when, as reaction feedstock, methylcyclopentane (MCP) as a five-membered ring compound and methylcyclohexane (MCH) as a hydrogen carrier were supplied to the reaction apparatus together with hydrogen, the performance of each of the corresponding catalyst samples was evaluated. As shown in Table 2, Examples 1-3 showed that, when MCP is used as the reaction feedstock, the hydrogen/MCP ratio (mol/mol) in the reaction feedstock was 28. Thus, when MCH is used as the reaction feedstock the hydrogen/MCH ratio (mol/mol) in the reaction feedstock is 28.

In Example 4, as a feedstock to be supplied to the hydrocracking apparatus 6, a mixture of 10 mol% methylcyclopentane as the five-membered ring compound and 90 mol% methylcyclohexane as the hydrogen carrier was prepared as a mixed feedstock, which was fed into the reaction apparatus together with hydrogen, and the performance of the catalyst sample was evaluated. As shown in Table 2, in Example 4, the hydrogen/(MCP + MCH) ratio (mol/mol) in the reaction feedstock is 28.

In Example 1-4, the hydrocracking reaction was caused at a mild temperature range where the catalyst layer temperature was approximately 200°C and Cobalt (Co) was used as active species to selectively promote the hydrocracking reaction that cleaves a carbon-carbon bond in five-membered ring compounds under the presence of hydrogen. Furthermore, a silica-based material exhibiting no solid acidity which could cause side reactions such as isomerization, was used as the catalyst carrier.

### (Example 1)

### Method of Preparing 3.0 wt%-Co/SiO₂ Catalyst

First, 0.2294 g cobalt nitrate hexahydrate (Co(NO₃)₂·6H₂O) and 75 mL pure water were added to a 300-mL-Erlenmeyer flask and thoroughly dissolved by ultrasonic agitation. Then, 1.5356 g amorphous silica powder (AEROSIL^{®} 380, Nippon Aerosil Co., Ltd., BET specific surface area 380 m²/g) was added as a carrier. The mixture was stirred at room temperature for 1 hour to impregnate and support the Co metal onto the silica at a loading rate of 3.0 wt%. After evaporating and drying at 40°C using an evaporator, the recovered product was transferred to an oven and dried overnight at 80°C to recover the powder. The powder was compressed, then properly crushed and sieved to produce particles with particle sizes of 500-600 µm. For reduction treatment of the catalyst, 1.2001 g of the sized catalyst was packed into a fixed-bed flow apparatus, and heated to 400°C over 30 minutes under nitrogen flow (30 mL/min), and the flow is then switched to hydrogen flow (30 mL/min) for one hour of reduction treatment. Subsequently, the reduced catalyst was cooled to room temperature while maintaining the hydrogen flow atmosphere, yielding a 3.0 wt%-Co/SiO₂ catalyst.

### Method for Testing Performance Evaluation of 3.0 wt%-Co/SiO₂ Catalyst

### [Hydrocracking Reaction of Methylcyclopentane (MCP) (Table 2)]

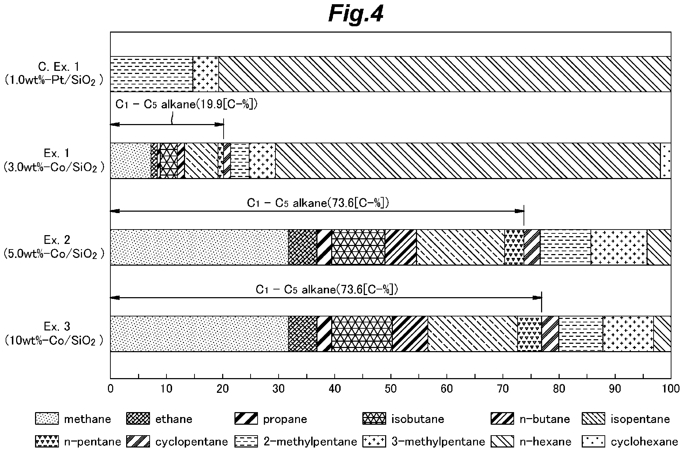

Tests for performance evaluation of the 3.0 wt%-Co/SiO₂ catalyst prepared by the above procedure were conducted according to the following steps. For catalyst pretreatment, 206.0 mg of the 3.0 wt%-Co/SiO₂ catalyst was charged into a fixed-bed circulating reactor, and heated to 400°C over 30 minutes under nitrogen flow (30 mL/min). Subsequently, the flow was switched to hydrogen flow (40 mL/min), and reduction treatment was performed for one hour. After the reduction treatment, the hydrocracking reaction of methylcyclopentane (MCP) was caused for a specified duration under the following conditions: hydrogen flow rate 40 mL/min, H₂/MCP = 28 mol/mol, W/F (ratio of catalyst amount to MCP flow rate) = 58.81 g-cat. h/mol, reaction temperature 200°C, total pressure 0.1 MPa. Five minutes after initiating the hydrocracking reaction, the product was sampled. The MCP conversion rate (%) and the reaction product selectivity (C-%) were determined by FID-GC (Flame Ionization Detector Gas Chromatography) analysis and used as indicators of catalyst performance (For the results of the performance evaluation test, see Table 3 indicated below and Figure 4).

### [Hydrocracking reaction of methylcyclohexane (MCH) (see Table 2)]

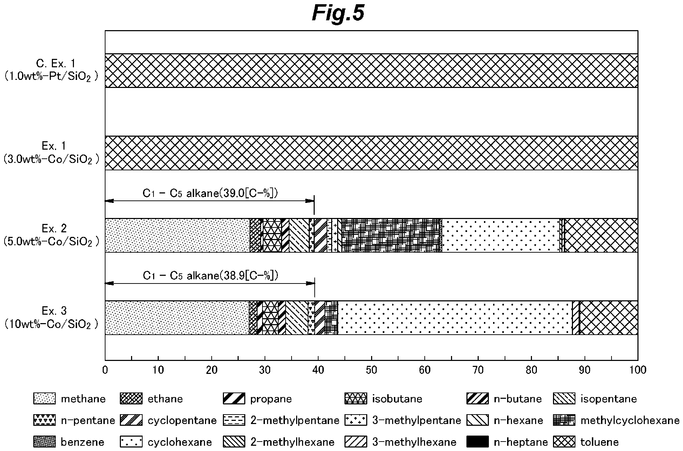

The tests for performance evaluation of the 3.0 wt%-Co/SiO₂ catalyst prepared by the above procedure were conducted according to the following steps. For catalyst pretreatment, 206.0 mg of the 3.0 wt%-Co/SiO₂ catalyst was charged into a fixed-bed circulating reactor, and was heated to 400°C over 30 minutes under nitrogen flow (30 mL/min). Subsequently, the flow was switched to hydrogen flow (30 mL/min), and reduction treatment was performed for one hour. After the reduction treatment, the hydrocracking reaction of methylcyclohexane (MCH) was caused for a specified duration under the following conditions: hydrogen flow rate 40 mL/min, H₂/MCH = 28 mol/mol, W/F (ratio of catalyst amount to MCH flow rate) = 58.77 g-cat. h/mol, reaction temperature 200°C, total pressure 0.1 MPa. Five minutes after initiating the hydrocracking reaction, the product was sampled. The MCH conversion rate (%) and the reaction product selectivity (C-%) were determined by FID-GC analysis and used as indicators of catalyst performance (For the results of the performance evaluation test, see Table 3 indicated below and Figure 5).

### (Example 2)

### Method of Preparing 5.0 wt%-Co/SiO₂ Catalyst

First, 0.3796 g cobalt nitrate hexahydrate (Co(NO₃)₂·6H₂O) and 75 mL pure water were added to a 300-mL-Erlenmeyer flask and thoroughly dissolved by ultrasonic agitation. Then, 1.5220 g amorphous silica powder (AEROSIL^{®} 380, Nippon Aerosil Co., Ltd., BET specific surface area 380 m²/g) was added as a carrier. The mixture was stirred at room temperature for 1 hour to impregnate and support the Co metal onto the silica at a loading rate of 5.0 wt%. After evaporating and drying at 40°C using an evaporator, the recovered product was transferred to an oven and dried overnight at 80°C to recover the powder. The powder was compressed, then properly crushed and sieved to produce particles with particle sizes of 500-600 µm. For reduction treatment of the catalyst, 1.2026 g of the sized catalyst was packed into a fixed-bed flow apparatus, and heated to 400°C over 30 minutes under nitrogen flow (30 mL/min), and the flow is then switched to hydrogen flow (30 mL/min) for five hours of reduction treatment. Subsequently, the reduced catalyst was cooled to room temperature while maintaining the hydrogen flow atmosphere, yielding a 5.0 wt%-Co/SiO2 catalyst.

### Method for Testing Performance Evaluation of 5.0 wt%-Co/SiO₂ Catalyst

### [Hydrocracking Reaction of Methylcyclopentane (MCP) (Table 2)]

Tests for performance evaluation of the 5.0 wt%-Co/SiO₂ catalyst prepared by the above procedure were conducted according to the following steps. For catalyst pretreatment, 210.0 mg of the 5.0 wt%-Co/SiO₂ catalyst was charged into a fixed-bed circulating reactor, and heated to 400°C over 30 minutes under nitrogen flow (30 mL/min). Subsequently, the flow was switched to hydrogen flow (40 mL/min), and reduction treatment was performed for one hour. After the reduction treatment, the hydrocracking reaction of methylcyclopentane (MCP) was caused for a specified duration under the following conditions: hydrogen flow rate 40 mL/min, H₂/MCP = 28 mol/mol, W/F (ratio of catalyst amount to MCP flow rate) = 59.95 g-cat. h/mol, reaction temperature 200°C, total pressure 0.1 MPa. Five minutes after initiating the hydrocracking reaction, the product was sampled. The MCP conversion rate (%) and the reaction product selectivity (C-%) were determined by FID-GC analysis and used as indicators of catalyst performance (For the results of the performance evaluation test, see Table 3 indicated below and Figure 4).

### [Hydrocracking reaction of methylcyclohexane (MCH) (see Table 2)]

The tests for performance evaluation of the 5.0 wt%-Co/SiO₂ catalyst prepared by the above procedure were conducted according to the following steps. For catalyst pretreatment, 210.0 mg of the 5.0 wt%-Co/SiO₂ catalyst was charged into a fixed-bed circulating reactor, and was heated to 400°C over 30 minutes under nitrogen flow (30 mL/min). Subsequently, the flow was switched to hydrogen flow (40 mL/min), and reduction treatment was performed for one hour. After the reduction treatment, the hydrocracking reaction of methylcyclohexane (MCH) was caused for a specified duration under the following conditions: hydrogen flow rate 40 mL/min, H₂/MCH = 28 mol/mol, W/F (ratio of catalyst amount to MCH flow rate) = 59.91 g-cat. h/mol, reaction temperature 200°C, total pressure 0.1 MPa. Five minutes after initiating the hydrocracking reaction, the product was sampled. The MCH conversion rate (%) and the reaction product selectivity (C-%) were determined by FID-GC analysis and used as indicators of catalyst performance (For the results of the performance evaluation test, see Table 3 indicated below and Figure 5).

### (Example 3)

### Method of Preparing 10 wt%-Co/SiO₂ Catalyst

First, 1.4944 g cobalt nitrate hexahydrate (Co(NO₃)₂·6H₂O) and 150 mL pure water were added to a 300-mL-Erlenmeyer flask and thoroughly dissolved by ultrasonic agitation. Then, 3.0182 g amorphous silica powder (AEROSIL^{®} 380, Nippon Aerosil Co., Ltd., BET specific surface area 380 m²/g) was added as a carrier. The mixture was stirred at room temperature for 1 hour to impregnate and support the Co metal onto the silica at a loading rate of 10 wt%. After evaporating and drying at 40°C using an evaporator, the recovered product was transferred to an oven and dried overnight at 80°C to recover the powder. The powder was compressed, then properly crushed and sieved to produce particles with particle sizes of 500-600 µm. For reduction treatment of the catalyst, 1.2753 g of the sized catalyst was packed into a fixed-bed flow apparatus, and heated to 400°C over 30 minutes under nitrogen flow (30 mL/min), and the flow is then switched to hydrogen flow (40 mL/min) for five hours of reduction treatment. Subsequently, the reduced catalyst was cooled to room temperature while maintaining the hydrogen flow atmosphere, yielding a 10 wt%-Co/SiO2 catalyst.

### Method for Testing Performance Evaluation of 10 wt%-Co/SiO₂ Catalyst

### [Hydrocracking Reaction of Methylcyclopentane (MCP) (Table 2)]

Tests for performance evaluation of the 10 wt%-Co/SiO₂ catalyst prepared by the above procedure were conducted according to the following steps. For catalyst pretreatment, 220.0 mg of the 10 wt%-Co/SiO₂ catalyst was charged into a fixed-bed circulating reactor, and heated to 400°C over 30 minutes under nitrogen flow (30 mL/min). Subsequently, the flow was switched to hydrogen flow (30 mL/min), and reduction treatment was performed for one hour. After the reduction treatment, the hydrocracking reaction of methylcyclopentane (MCP) was caused for a specified duration under the following conditions: hydrogen flow rate 40 mL/min, H₂/MCP = 28 mol/mol, W/F (ratio of catalyst amount to MCP flow rate) = 62.80 g-cat. h/mol, reaction temperature 200°C, total pressure 0.1 MPa. Five minutes after initiating the hydrocracking reaction, the product was sampled. The MCP conversion rate (%) and the reaction product selectivity (C-%) were determined by FID-GC analysis and used as indicators of catalyst performance (For the results of the performance evaluation test, see Table 3 indicated below and Figure 4).

### [Hydrocracking reaction of methylcyclohexane (MCH) (see Table 2)]

The tests for performance evaluation of the 10 wt%-Co/SiO₂ catalyst prepared by the above procedure were conducted according to the following steps. For catalyst pretreatment, 220.0 mg of the 10 wt%-Co/SiO₂ catalyst was charged into a fixed-bed circulating reactor, and was heated to 400°C over 30 minutes under nitrogen flow (30 mL/min). Subsequently, the flow was switched to hydrogen flow (40 mL/min), and reduction treatment was performed for one hour. After the reduction treatment, the hydrocracking reaction of methylcyclohexane (MCH) was caused for a specified duration under the following conditions: hydrogen flow rate 40 mL/min, H₂/MCH = 28 mol/mol, W/F (ratio of catalyst amount to MCH flow rate) = 62.76 g-cat. h/mol, reaction temperature 200°C, total pressure 0.1 MPa. Five minutes after initiating the hydrocracking reaction, the product was sampled. The MCH conversion rate (%) and the reaction product selectivity (C-%) were determined by FID-GC analysis and used as indicators of catalyst performance (For the results of the performance evaluation test, see Table 3 indicated below and Figure 5).

### (Example 4)

### Method of Preparing 10 wt%-Co/SiO₂ Catalyst

The same method as that of Example 3 was conducted to yield 10 wt%-Co/SiO2 catalyst.

### Method for Testing Performance Evaluation of 10 wt%-Co/SiO₂ Catalyst

### [Hydrocracking Reaction of Mixture of Methylcyclopentane (MCP) and Methylcyclohexane (MCH) (Table 2)]

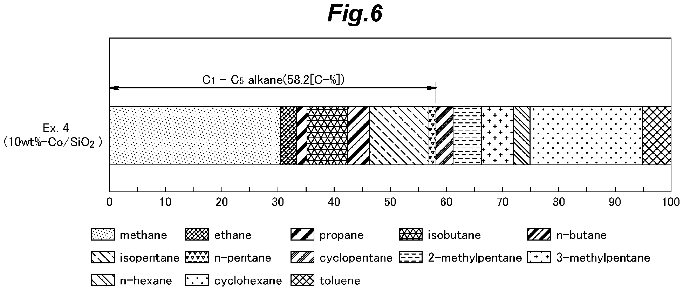

Tests for performance evaluation of the 10 wt%-Co/SiO₂ catalyst prepared by the above procedure were conducted according to the following steps. For catalyst pretreatment, 220.0 mg of the 10 wt%-Co/SiO₂ catalyst was charged into a fixed-bed circulating reactor, and heated to 400°C over 30 minutes under nitrogen flow (30 mL/min). Subsequently, the flow was switched to hydrogen flow (40 mL/min), and reduction treatment was performed for one hour. After the reduction treatment, the hydrocracking reaction of a mixture of methylcyclopentane (MCP) and methylcyclohexane (MCH) was caused for a specified duration under the following conditions: hydrogen flow rate 40 mL/min, H₂/(MCP+MCH) = 28 mol/mol, H₂ : MCP : MCH = 28 : 0.1 : 0.9, W/F (ratio of catalyst amount to (MCP+MCH) flow rate) = 67.95 g-cat. h/mol, reaction temperature 200°C, total pressure 0.1 MPa. Five minutes after initiating the hydrocracking reaction, the product was sampled. The MCP-and-MCH conversion rate (%) and the reaction product selectivity (C-%) were determined by FID-GC analysis and used as indicators of catalyst performance (For the results of the performance evaluation test, see Table 4 indicated below and Figure 6).

### (Comparative Example 1)

### Method of Preparing 1.0 wt%-Pt/SiO₂ Catalyst

A commercially available silica-supported platinum catalyst (1.0 wt%-Pt/SiO₂) (Sigma-Aldrich, 520691-25G) was used as a catalyst sample (Table 1).

### Method for Testing Performance Evaluation of 1.0 wt%-Pt/SiO₂ Catalyst

### [Hydrocracking Reaction of Methylcyclopentane (MCP) (Table 2)]

Tests for performance evaluation of the above-described commercially available product (1.0 wt%-Pt/SiO₂ catalyst). For catalyst pretreatment, 101.0 mg of the 1.0 wt%-Pt/SiO₂ catalyst was charged into a fixed-bed circulating reactor, and heated to 400°C over 30 minutes under nitrogen flow (30 mL/min). Subsequently, the flow was switched to hydrogen flow (40 mL/min), and reduction treatment was performed for one hour. After the reduction treatment, the hydrocracking reaction of methylcyclopentane (MCP) was caused for a specified duration under the following conditions: hydrogen flow rate 40 mL/min, H₂/MCP = 28 mol/mol, W/F (ratio of catalyst amount to MCP flow rate) = 28.83 g-cat. h/mol, reaction temperature 200°C, total pressure 0.1 MPa. Five minutes after initiating the hydrocracking reaction, the product was sampled. The MCP conversion rate (%) and the reaction product selectivity (C-%) were determined by FID-GC analysis and used as indicators of catalyst performance (For the results of the performance evaluation test, see Table 3 indicated below and Figure 4).

### [Hydrocracking reaction of methylcyclohexane (MCH) (see Table 2)]

The tests for performance evaluation of the above-described commercially available product (1.0 wt%-Pt/SiO₂ catalyst). For catalyst pretreatment, 101.0 mg of the 3.0 wt%-Co/SiO₂ catalyst was charged into a fixed-bed circulating reactor, and was heated to 400°C over 30 minutes under nitrogen flow (30 mL/min). Subsequently, the flow was switched to hydrogen flow (40 mL/min), and reduction treatment was performed for one hour. After the reduction treatment, the hydrocracking reaction of methylcyclohexane (MCH) was caused for a specified duration under the following conditions: hydrogen flow rate 40 mL/min, H₂/MCH = 28 mol/mol, W/F (ratio of catalyst amount to MCH flow rate) = 28.81 g-cat. h/mol, reaction temperature 200°C, total pressure 0.1 MPa. Five minutes after initiating the hydrocracking reaction, the product was sampled. The MCH conversion rate (%) and the reaction product selectivity (C-%) were determined by FID-GC analysis and used as indicators of catalyst performance (For the results of the performance evaluation test, see Table 3 indicated below and Figure 5).

Table 3 indicates the results of the performance evaluation tests for the respective catalysts of Examples 1-3 and Comparative Example 1. Figure 4 shows the distribution of reaction products in the hydrocracking of MCP obtained in the performance evaluation tests of the respective catalysts of Examples 1-3 and Comparative Example 1. Figure 5 shows the distribution of reaction products in the hydrocracking of MCH, as in Figure 4, obtained in the performance evaluation tests of the respective catalysts. In the performance evaluation tests of the respective catalysts, methylcyclopentane (MCP) and methylcyclohexane (MCH) were independently used as a single reaction feedstock, and made to undergo hydrocracking reactions under the same conditions.

**[Table 3]**

| | catalyst | FS: (MCP+H₂) (H₂/MCP =28 [mol/mol] | FS: (MCH+H₂) (H₂/MCH =28 [mol/mol]) |
|---|---|---|---|
| | | MCP conv rate [%] | MCH conv rate [%] |
| C. Ex. 1 | 1.0wt%-Pt/SiO₂ | < 1 | < 3 |
| Ex. 1 | 3.0wt%-Co/SiO₂ | 1.9 | < 1 |
| Ex. 2 | 5.0wt%-Co/SiO₂ | 20.5 | < 1 |
| Ex. 3 | 10wt%-Co/SiO₂ | 32.7 | < 1 |

As shown in Table 3, in Comparative Example 1 (1.0 wt%-Pt/SiO₂ catalyst), when MCP was used as the feedstock, hydrocracking reaction occurred scarcely, and the conversion rate of MCP was less than 1%. The reaction products when MCP was used as the feedstock were direct ring-opening products: n-hexane, 2-methylpentane, and 3-methylpentane, as shown in Figure 4. As shown in Table 3, when MCH was used as the feedstock, the conversion rate of MCH was less than 3%. The reaction products when MCH was used as the feedstock were all toluene (TOL), as shown in Figure 5.

Results of Comparative Example 1 show that, when platinum was used as the active species supported on silica, the catalyst exhibited activity in the dehydrogenation-aromatization reaction of cyclohexanes (The reaction that produces TOL by dehydrogenation of MCH in this case), but almost no activity in the hydrocracking reaction of cyclopentanes (MCP, in this case).

The catalyst of Example 1 (3.0 wt%-Co/SiO₂ catalyst) exhibited activity in the hydrocracking reaction of MCP, with an MCP conversion rate of approximately 2% (Table 3). When MCP was used as the feedstock, the reaction products comprised approximately 20% C₁-C₅ alkanes (i.e., light alkanes with 1-5 carbons), which are easily separable in subsequent processes, and the reminder comprised direct ring-opening products: n-hexane, 3-methylpentane, and 2-methylpentane, and naphthenes such as cyclopentane and cyclohexane (Figure 4). When MCH was used as the feedstock, hydrocracking reactions occurred to a negligible extent, with an MCH conversion rate below 1% (Table 3), and the reaction product was entirely toluene (TOL) (Figure 5).

The catalyst of Example 2 (5.0 wt%-Co/SiO₂) showed improved activity in the hydrocracking reaction of MCP compared to Example 1, with an MCP conversion rate of 20.5% (Table 3). When MCP was used as the feedstock, the reaction products comprised 73.6% C₁-C₅ alkanes (i.e., light alkanes with 1-5 carbons), and the reminder comprised direct ring-opening products (i.e., n-hexane, 3-methylpentane, and 2-methylpentane), and naphthenes (i.e., cyclopentane and cyclohexane) (Figure 4). When MCH was used as the feedstock, hydrocracking reactions occurred to a negligible extent, similar to Example 1, and the MCH conversion rate was below 1% (Table 3). The reaction products when MCH was used as the feedstock contained 39% C₁-C₅ alkanes (Figure 5).

The catalyst of Example 2 (5.0 wt%-Co/SiO₂) showed further improved activity in the hydrocracking reaction of MCP compared to Examples 1 and 2, achieving the MCP conversion rate of 32.7% (Table 3). When MCP was used as the feedstock, the reaction products comprised 76.8% C₁-C₅ alkanes (i.e., light alkanes with 1-5 carbons), and the reminder comprised direct ring-opening products (i.e., n-hexane, 3-methylpentane, and 2-methylpentane), and naphthenes (i.e., cyclopentane and cyclohexane) (Figure 4). When MCH was used as the feedstock, hydrocracking reactions occurred to a negligible extent, similar to Examples 1 and 2, and the MCH conversion rate was below 1% (Table 3). The reaction products when MCH was used as the feedstock contained 39% C₁-C₅ alkanes (Figure 5).

The results of Example 1-3 show that cobalt supported on amorphous silica as the active species exhibits high decomposition activity and high selectivity toward C₁-C₅ alkanes in the hydrocracking reaction of cyclopentanes, while its activity in the hydrocracking reaction of cyclohexanes is low.

Example 1-3 show metal catalysts (i.e., hydrocracking catalysts) used in the hydrocracking reaction of cyclopentanes, containing 3.0 wt%, 5.0 wt%, and 10 wt% cobalt, respectively. However, cobalt can be expected to achieve similar effects within the range of 1.0 wt% to 50 wt%. Particularly, in order to effectively enhance selectivity toward C₁-C₅ alkanes, the hydrocracking catalyst desirably contains at least 5 wt% or more of cobalt as the active species.

Example 4 used the 10 wt% Co/SiO₂ catalyst (Example 3) that demonstrated the best performance in Examples 1-3 and caused hydrocracking of a mixed feedstock of MCP and MCH, making the process closer to reality. Table 4 shows results of the performance evaluation test of catalysts used in Example 4. Figure 6 shows the distribution of reaction products in the hydrocracking of a mixture of MCP and MCH (MCP+MCH) obtained by performance evaluation test of catalysts used in Example 4.

**[Table 4]**

| | catalyst | FS: (MCP+MCH)+H₂ (H₂/(MCP+MCH) =30 [mol/mol] , H₂:MCP:MCH=28:0.1:0.9) | | |
|---|---|---|---|---|
| | | (MCP+MCH) conv rate [%] | MCP conv rate [%] | MCH conv rate [%] |
| Ex. 4 | 10wt%-Co/SiO₂ | 16.6 | 13.2 | 3.4 |

As shown in Table 4, the tests of Example 4 showed the overall conversion rate for the MCP+MCH mixed feedstock was 16.6%, of which the MCP conversion rate was 13.2% and the MCH conversion rate was 3.4%.

These test results confirmed that the 10 wt%-Co/SiO₂ catalyst selectively hydrocracked MCP while suppressing the hydrocracking of MCH in the performance evaluation tests using a mixture of MCP and MCH, simulating actual use in the hydrogen storage and transportation system 1 shown in Figure 1, as well.

Furthermore, as shown in Figure 6, it was also confirmed that even when MCP + MCH was used as the feedstock, the reaction product contained 58.2% C₁-C₅ alkanes, the decomposition into light alkanes proceeded with high selectivity.

Thus, the results of Examples 1-4 (Tables 3 and 4 and Figures 4-6) revealed that using a metal catalyst with cobalt supported on a silica-based carrier such as amorphous silica enables the highly selective hydrocracking of cyclopentanes contained in methylcyclohexane into light alkanes.

The present invention has been described in terms of specific embodiments. However, it is not limited to such embodiments, and can be embodied with various modifications. In the metal catalysts and methods for hydrocracking a cyclic compound using the same according to the above-described embodiments, not all elements included therein are essential, and some of them may be eliminated or replaced as appropriate.

For example, where to use the metal catalysts and the method for hydrocracking cyclic compounds using the same (according to the present invention) is not limited to the hydrocracking apparatus 6 provided in the hydrogen storage and transportation system 1 described above. The metal catalyst and the method for hydrocracking a cyclic compound using the same according to the present invention can be widely used for applications which involve the hydrocracking of a cyclic compound that has a five-membered ring structure and is included in a saturated cyclic compound.

### GLOSSARY

- 1: hydrogen storage and transportation system
- 2: hydrogenation plant
- 3: dehydrogenation plant
- 4: storage facility
- 5: storage facility
- 6: hydrocracking apparatus
- 11: hydrogenation reactor
- 12: MCH storage tank
- 13: TOL storage tank
- 21: dehydrogenation reactor
- 22: TOL storage tank
- 23: MCH storage tank

## Claims

1. A metal catalyst used for hydrocracking a cyclic compound that has a five-membered ring structure and is included in a saturated cyclic compound used as a hydrogen carrier, the metal catalyst comprising:
a silica-based support serving as a catalyst carrier, and
cobalt as a metal supported on the catalyst carrier.

2. The metal catalyst as claimed in claim 1, wherein the metal catalyst comprises cobalt as the metal in an amount ranging from 1 wt% to 50 wt%.

3. The metal catalyst as claimed in claim 2, wherein the metal catalyst comprises cobalt as the metal in an amount of 5 wt% or more.

4. The metal catalyst as claimed in claim 1 or 2, wherein the silica-based support comprises amorphous silica.

5. A method for hydrocracking a cyclic compound using the metal catalyst as claimed in any one of claims 1 to 3, wherein the cyclic compound has a five-membered ring structure and is included in a saturated cyclic compound used as a hydrogen carrier, the method comprising:
causing dehydrogenation of the saturated cyclic compound to generate an aromatic compound and the cyclic compound having the five-membered ring structure, as an impurity of the aromatic compound;
causing hydrogenation of the aromatic compound containing the impurity, thereby generating a mixture of the saturated cyclic compound and the impurity; and
hydrocracking the mixture of the saturated cyclic compound and the impurity using the metal catalyst.

6. The method as claimed in claim 5, wherein the step of hydrocracking comprises:
supplying the mixture of the saturated cyclic compound and the impurity together with 50 to 99.8 mol% hydrogen to the metal catalyst having a catalyst layer, while a temperature of the catalyst layer is maintained at 150°C to 300°C.
